# EUROPÄISCHE PATENTSCHRIFT

(11) **EP 0 527 258 B1**
(45) Veröffentlichungstag und Bekanntmachung des Hinweises auf die Patenterteilung: **25.10.1995**
(21) Anmeldenummer: 91113661.2
(22) Anmeldetag: 14.08.1991
(51) Int. Cl.: G01N 27/12, G01N 33/00

(54) **Gassensor-Array zur Detektion einzelner Gaskomponenten in einem Gasgemisch**
Gas sensor array for the detection of individual gas components in a gas mixture
Matrice de détecteurs de gaz pour la détection des composants individuels dans un mélange de gaz

(43) Veröffentlichungstag der Anmeldung: 17.02.1993
(73) Patentinhaber: SIEMENS AKTIENGESELLSCHAFT, 80333 München (DE)
(72) Erfinder: Fleischer, Maximilian, Dipl.-Phys., W-8000 München 19 (DE); Meixner, Hans, Dr. rer. nat., W-8013 Haar (DE)

(56) Entgegenhaltungen:
- EP-A- 0 141 090
- FR-A- 2 331 016
- GB-A- 1 562 623
- US-A- 4 457 161
- US-A- 4 574 264

## Beschreibung

Die vorliegende Erfindung betrifft ein Gassensor-Array zur Detektion einzelner Gaskomponenten in einem Gasgemisch, bestehend aus einer Vielzahl von Einzelsensorelementen auf der Basis halbleitender Metalloxide, wobei die Einzelsensorelemente jeweils auf ein elektrisch nichtleitendes Substrat aufgebracht sind, wobei das Array mit einer Kontaktelektroden-Anordnung zum Messen der elektrischen Leitfähigkeiten, einer Heizanordnung zum Heizen bei einer vorbestimmten Betriebstemperatur, einer Schutzhülle, die das Array vor äußeren mechanischen Einflüssen schützt, und einem Befestigungssockel versehen ist, wobei den Einzelsensorelementen vorgegebene, individuelle Betriebstemperaturen zugeordnet sind und wobei zur Detektion der einzelnen Gaskomponenten Kombinationen zwischen den jeweiligen Sensorsignalen gebildet werden, die einer Verarbeitungseinheit zuzuführen sind.

Zur selektiven Detektion und Quantifizierung einzelner Komponenten in einem Gasgemisch aus chemisch unterschiedlichen Gasen sind bisher zwei Vorschläge bekannt geworden. Einer dieser Vorschläge betrifft sog. Analysengeräte, bei denen beispielsweise die Verwendung für die Quadrupolmassenspektroskopie oder "FTIR" vorgesehen sind. Diese relativ aufwendigen Apparaturen besitzen die erforderliche Eignung für Meßaufgaben an Versuchsständen, wie z. B. Motorprüfständen und Gasmeßplätzen. Auf dem Gebiet der sog. Niedrigkosten-Geräte, die für Überwachungsaufgaben verwendet werden können, ist vorgeschlagen worden, Gassensoren auf der Grundlage geheizter Röhrchen aus halbleitendem SnO₂, das mit unterschiedlichen Edelmetalldotierungen versehen ist, um ansatzweise die Selektivität eines Sensors für ein bestimmtes Gas zu erhalten, zu verwenden, vgl. z. B. J. Watson, A. Price, Proc. IEEE 66, 1670 (1978) betreffend eine Untersuchung der Selektivität derartiger Sensoren bezüglich CO und CH₄.

Die zuletzt genannten Sensoren weisen starke Querempfindlichkeiten auf, d. h. sie sind im allgemeinen nicht nur für das zu detektierende Gas sensitiv. So ist z. B. bei Sensoren für reduzierende Gase auf der Basis SnO₂ zu beachten, daß das Grundmaterial SnO₂ selbst auch O₂-sensitiv ist, d. h. daß diese Sensoren für reduzierende Gase auch auf O₂ reagieren. Auswertungsverfahren nach dem Prinzip einer Mustererkennung (pattern recognition) sind mit diesen Sensoren nur bedingt durchzuführen, da die Einzelsensoren eines betreffenden Array die hierfür benötigte Stabilität im allgemeinen nicht aufweisen. Die sich ergebenden Probleme liegen in einer Drift des Sensorsignals und der Exemplarstreuung begründet, vgl. dazu z. B. S. R. Morrison, Gas Sensing with Solid State Devices, Acadamic Press, New York, 1989, Kapitel 13.1.2.

Ähnlich gelagerte Probleme ergeben sich auch bei anderen gebräuchlichen Sensormaterialien, wie z. B. Fe₂O₃, TiO₂ und besonders bei ZnO.

Der vorliegenden Erfindung liegt die Aufgabe zugrunde, ein Gassensor-Array zur Detektion einzelner Gaskomponenten in einem Gasgemisch zu schaffen, das mit hoher Zuverlässigkeit und geringer Querempfindlichkeit eine Detektion und Quantifizierung einzelner Komponenten in einem Gasgemisch aus chemisch unterschiedlichen Gasen ermöglicht, wobei das Gassensor-Array einen kostengünstigen Aufbau aufweisen soll.

Die genannte Aufgabe wird durch ein Gassensor-Array der eingangs genannten Art und gemäß dem Oberbegriff des Patentanspruchs 1 gelöst, das erfindungsgemäß durch das im kennzeichnenden Teil des Patentanspruchs 1 angegebene Merkmal bestimmt ist.

Vorteilhafte Weiterbildungen der Erfindung sind durch die in den Unteransprüchen angegebenen Merkmale gekennzeichnet.

Die Erfindung gestattet die Verwendung von Arrays, die einzelne Sensorelemente auf der Basis katalytisch nicht aktiver, stabiler halbleitender Dünnschichten, z. B. Ga₂O₃, enthalten, wobei bei den einzelnen Sensorelementen
- durch Wahl der Betriebstemperatur zwischen Sauerstoffsensivität und Sensitivität für reduzierende Gase gewählt wird,
- einzelnen Sensorelementen durch gezielte katalytische Aktivierung des selbst nicht katalytisch aktiven Ga₂O₃ und Wahl einer geeigneten Betriebstemperatur eine für einzelne reduzierende Gase spezifische Sensitivität verliehen wird und
- einer Einstellung der Sensitivität für oxidierende (NOₓ, CL₂, Br₂) Gase durch Änderung des Typs der Halbleitung (Wechsel zu p-Typ) mittels Dotierung des Materials möglich ist.

Im folgenden wird die Erfindung anhand mehrerer Figuren im einzelnen beschrieben.
- Fig. 1: zeigt beispielhaft den Aufbau eines Einzelsensorelements, wie es in dem erfindungsgemäßen Gassensor-Array verwendet werden kann.

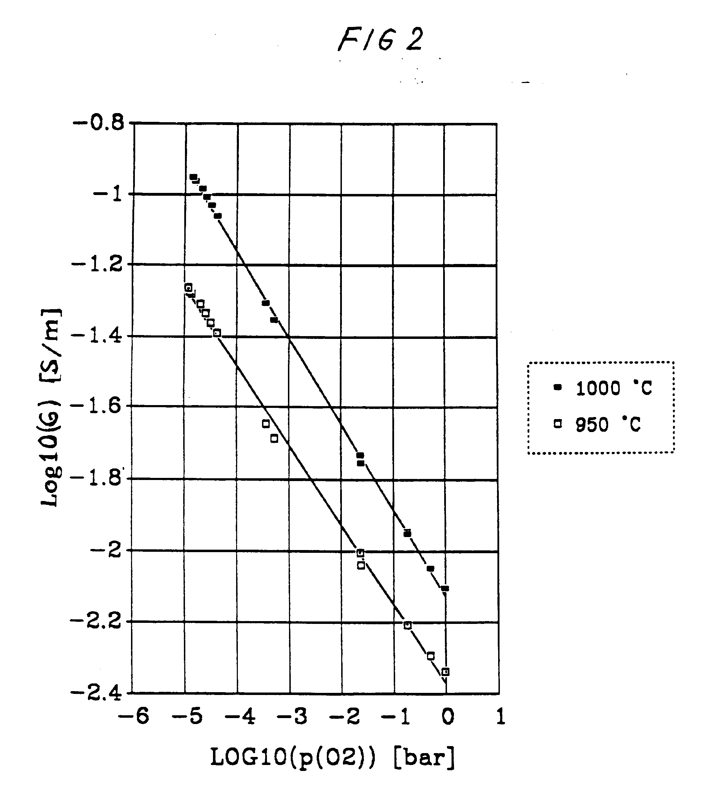

- Fig. 2: zeigt die Sensorkennlinien eines Ga₂O₃-Dünnschicht-Sauerstoffsensorelements für O₂ bei verschiedenen Betriebstemperaturen.

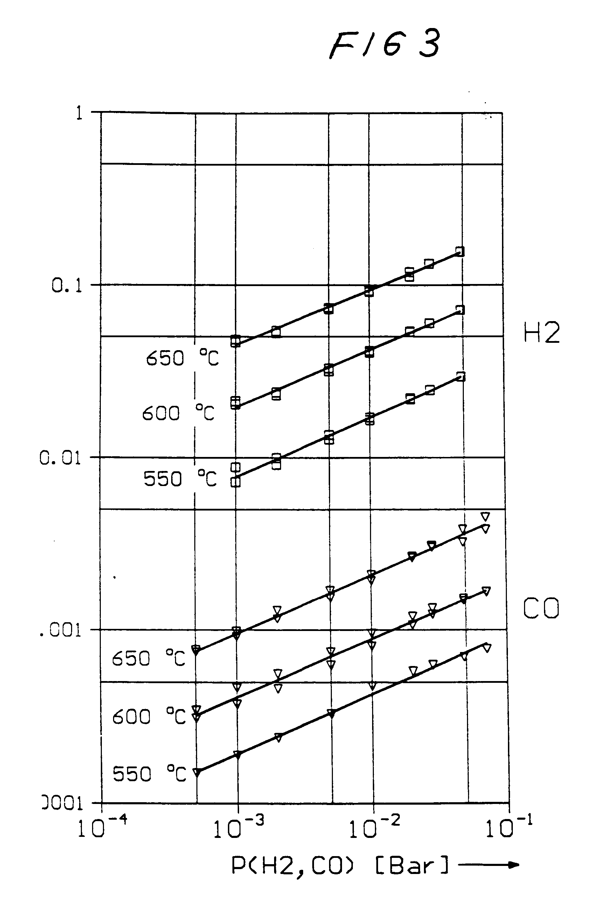

- Fig. 3: zeigt die Sensorkennlinien eines Ga₂O₃-Gassensorelements zur Detektion von H₂ und Co bei verschiedenen Betriebstemperaturen.

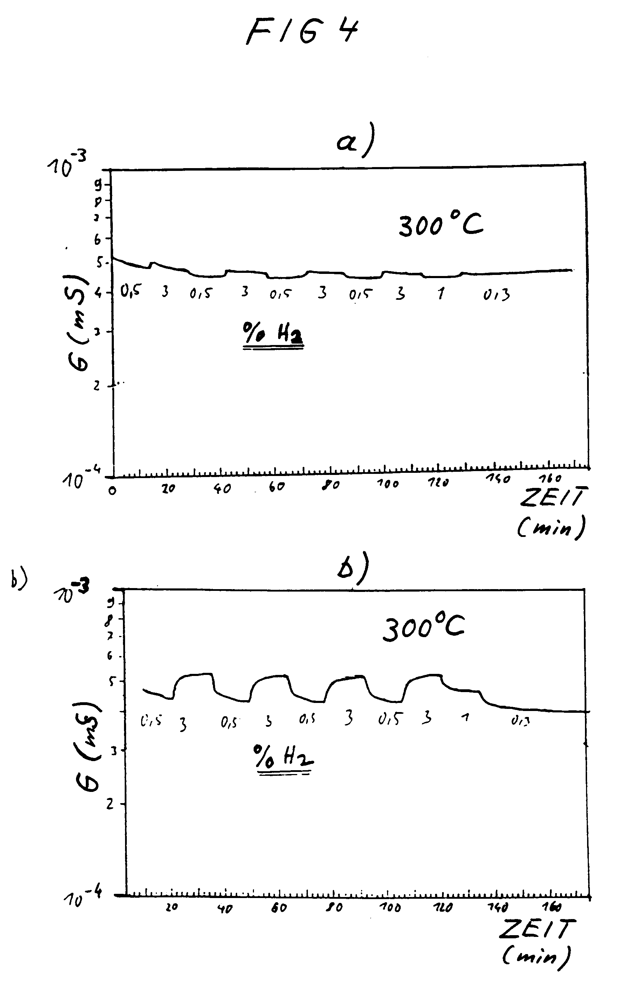

- Fig. 4: zeigt beispielhaft die Wirkung einer katalytischen Aktivierung eines Ga₂O₃-Gassensors.
- Fig. 5: zeigt ein Ausführungsbeispiel einer Sensoranordnung zur Unterscheidung von O₂ und reduzierenden Gasen sowie einen typischen Temperaturverlauf über die Anordnung.
- Fig. 6: zeigt ein weiteres Ausführungsbeispiel zur Erzielung verschiedener Temperaturbereiche in einem Gassensor-Array.
- Fig. 7: zeigt eine schematische Querschnittsdarstellung eines Gassensor-Array zur quantitativen Detektion einzelner Gaskomponenten.
- Fig. 8: zeigt ein weiteres Ausführungsbeispiel zur Erzielung verschiedener Temperaturbereiche in einem Gassensor-Array.

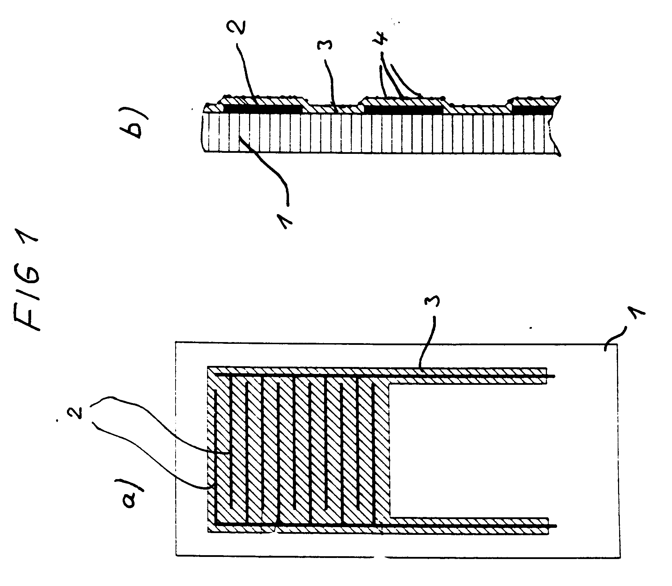

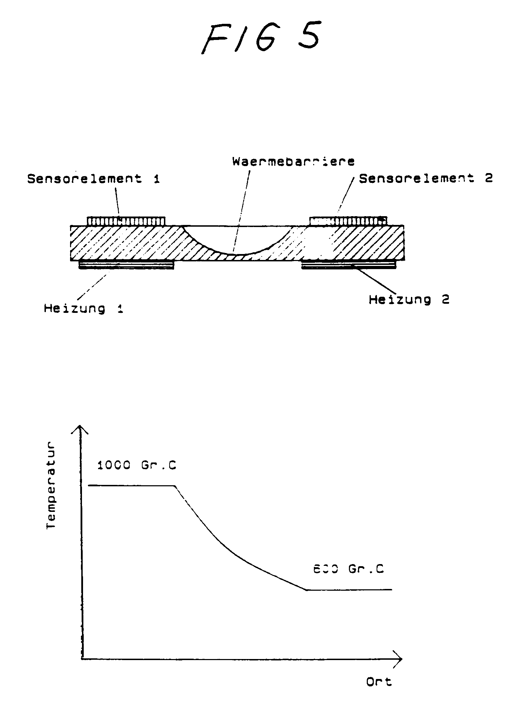

Die vorliegende Erfindung geht von einem Gassensor-Array zur Detektion einzelner Gaskomponenten in einem Gasgemisch aus, das aus einer Vielzahl von Einzelsensorelementen auf der Basis halbleitender Metalloxide besteht, wobei die Einzelsensorelemente jeweils auf ein elektrisch nichtleitendes Substrat 1 aufgebracht sind. Das Array ist mit einer Kontaktelektroden-Anordnung 2 zum Messen der elektrischen Leitfähigkeiten, vgl. Fig. 1a, einer Heizanordnung zum Heizen bei einer vorbestimmten Betriebstemperatur, vgl. Fig. 5, einer Schutzhülle (nicht gezeigt), die das Array vor äußeren mechanischen Einflüssen schützt, und einem Befestigungssockel (nicht gezeigt) versehen, wobei den Einzelsensorelementen vorgegebene, individuelle Betriebstemperaturen zugeordnet sind und wobei zur Detektion der einzelnen Gaskomponenten Kombinationen aus den jeweiligen Sensorsignalen gebildet werden, die einer Verarbeitungseinheit zuzuführen sind. Erfindungsgemäß basiert zumindest ein Teil der Einzelsensorelemente auf β-Ga₂O₃ Dünnschichten 3. Alle Einzelsensorelemente sind auf einem gemeinsamen Substrat in planarer Anordnung vorgesehen. Zumindest eines der Einzelsensorelemente ist mit zumindest einem temperaturstabilen, fein dispergierten, katalytisch aktiven Material 4, vgl. Fig. 1b, versehen, das in seiner Wirkung bei den betreffenden herrschenden Umgebungsbedingungen stabil ist. Das katalytisch aktive Material 4 kann ein Edelmetall oder eine Legierung mit zumindest einem Edelmetall sein. Als Edelmetall kann zweckmäßigerweise Pt, Pd, Rh oder Ag verwendet werden.

Erfindungsgemäß ist auch vorgesehen, daß das katalytisch aktive Material ein Nebengruppen-Metalloxid ist. Das Nebengruppenmetalloxid kann beispielsweise Nb₂O₃, Fe₂O₃, ZrO₂ oder TiO₂ sein.

Für die Herstellung des erfindungsgemäßen Gassensor-Array ist vorgesehen, daß das katalytisch aktive Material auf die Oberfläche der gassensitiven Ga₂O₃-Dünnschicht in Form einer zweiten Phase aufgebracht wird. Das katalytisch aktive Material kann sich auch in Form einer zweiten Phase innerhalb der gassensitiven Ga₂O₃-Dünnschicht befinden.

Es ist auch vorgesehen, daß bei zumindest einem der Einzelsensorelemente die ursprünglich n-halbleitende Ga₂O₃-Dünnschicht 3 zur Erfassung oxidierender Gase durch Dotierung zu einer p-halbleitenden Dünnschicht umgewandelt ist. Das Dotierungsmaterial ist vorzugsweise MgO.

Die Einzelsensorelemente können unterschiedliche Schichtmorphologien aufweisen.

Erfindungsgemäß ist außerdem vorgehen, daß die Einzelsensorelemente in ihrem Aufbau je nach der zu erfassenden Gaskomponente oder je nach der zu erfassenden Gaskomponentengruppe unterschiedlich sind. Die Betriebstemperaturen können dabei für die Einzelsensorelemente gleich sein. Ein Ausführungsbeispiel sieht darüber hinaus vor, daß zumindest einem Teil der Vielzahl von Einzelsensorelementen unterschiedliche unterschiedliche Betriebstemperaturen zugeordnet sind. Die können jedoch auch einen gleichen Aufbau aufweisen, wobei den Einzelsensorelementen unterschiedliche Betriebstemperaturen zugeordnet sind.

Die unterschiedlichen Betriebstemperaturen sind je nach der zu erfassenden Gaskomponente oder nach der zu erfassenden Gassensor-Komponentengruppe vorgegeben, z. B. 950 °C für O₂ und 600 °C für reduzierende Gase.

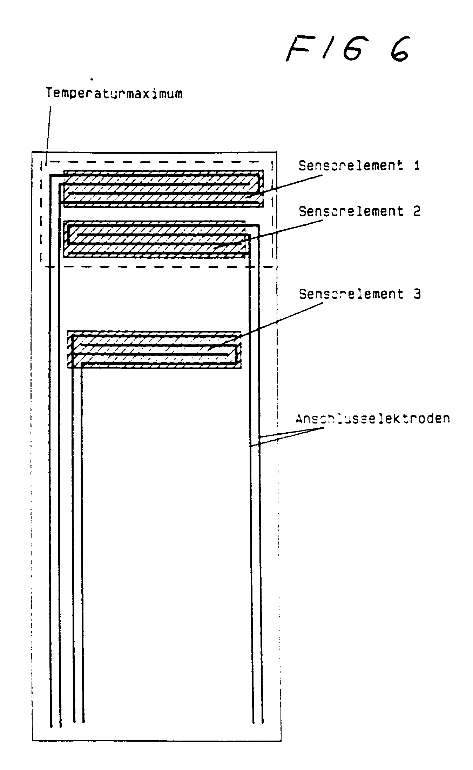

Auf der sensorabgewandten Seite des Trägerkörpers ist eine Heizstruktur vorgesehen, die im Zusammenwirken mit im wesentlichen der Wärmesenke "Befestigungssockel" einen Temperaturgradienten im Trägerkörper erzeugt, in dessen Bereich die Einzelsensorelemente angeordnet sind, so daß die unterschiedlichen Betriebstemperaturen bereitgestellt sind, vgl. Fig. 6.

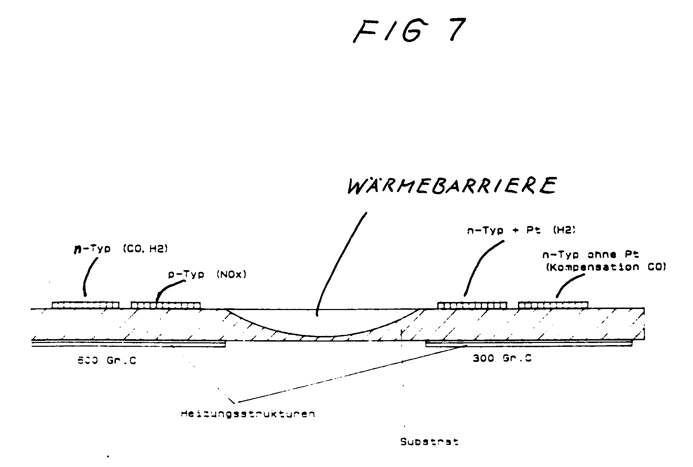

Es sind gemäß einem Ausführungsbeispiel zumindest durch eine oder mehrere Wärmebarrieren thermisch im wesentlichen isolierte Bereiche des Trägerkörpers vorgesehen. Jedem dieser Bereiche ist eine eigene Heizstruktur zugeordnet, so daß die unterschiedlichen Betriebstemperaturen bereitgestellt sind, vgl. Fig. 5 oder Fig. 7. Die Heizstrukturen können in Reihe oder parallel geschaltet sein.

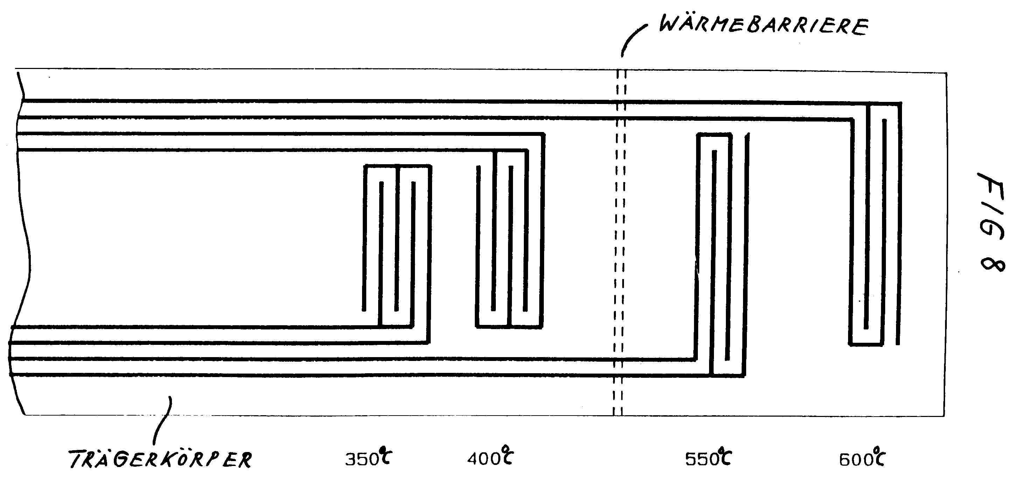

Die unterschiedlichen Betriebstemperaturen können durch eine Struktur, die sich aus einer Kombination der zuvor genannten Anordnungen ergibt, erzeugt werden. Die Wärmebarriere kann beispielsweise in Form einer Einsenkung in dem Trägerkörper oder in Form eines Spalts in dem Trägerkörper ausgebildet sein, vgl. Fig. 8.

Der Trägerkörper kann beispielsweise aus einem Keramikmaterial oder aus Quarzglas bestehen. Es kann jedoch auch vorgesehen sein, daß der Trägerkörper aus Silizium mit zumindest einer elektrisch isolierenden Schicht besteht.

## Patentansprüche

1. Gassensor-Array zur Detektion einzelner Gaskomponenten in einem Gasgemisch, bestehend aus einer Vielzahl von Einzelsensorelementen auf der Basis halbleitender Metalloxide, wobei die Einzelsensorelemente jeweils auf ein elektrisch nichtleitendes Substrat aufgebracht sind, wobei das Array mit einer Kontaktelektroden-Anordnung zum Messen der elektrischen Leitfähigkeiten, einer Heizanordnung zum Heizen bei einer vorbestimmten Betriebstemperatur, einer Schutzhülle, die das Array vor äußeren mechanischen Einflüssen schützt, und einem Befestigungssockel versehen ist, wobei den Einzelsensorelementen vorgegebene, individuelle Betriebstemperaturen zugeordnet sind und wobei zur Detektion der einzelnen Gaskomponenten Differenzen zwischen den jeweiligen Sensorsignalen gebildet werden, die einer Verarbeitungseinheit zuzuführen sind,
dadurch **gekennzeichnet,**
daß zumindest ein Teil der Einzelsensorelemente auf β-Ga₂O₃-Dünnschichten (3) basiert und daß alle Einzelsensorelemente auf einem gemeinsamen Substrat (1) in planarer Anordnung vorgesehen sind.

2. Gassensor-Array nach Anspruch 1,
**dadurch gekennzeichnet,**
daß zumindest eines der Einzelsensorelemente mit zumindest einem fein dispergierten, katalytisch aktiven Material (4) versehen ist, das in seiner Wirkung bei den betreffenden herrschenden Umgebungsbedingungen stabil ist.

3. Gassensor-Array nach Anspruch 2,
dadurch **gekennzeichnet,**
daß das katalytisch aktive Material (4) ein Edelmetall ist.

4. Gassensor-Array nach Anspruch 3,
dadurch **gekennzeichnet,**
daß das katalytisch aktive Material (4) eine Legierung mit zumindest einem Edelmetall ist.

5. Gassensor-Array nach Anspruch 3,
dadurch **gekennzeichnet,**
daß das Edelmetall Pt ist.

6. Gassensor-Array nach Anspruch 3,
dadurch **gekennzeichnet,**
daß das Edelmetall Pd ist.

7. Gassensor-Array nach Anspruch 3,
dadurch **gekennzeichnet,**
daß das Edelmetall Rh ist.

8. Gassensor-Array nach Anspruch 3,
dadurch **gekennzeichnet,**
daß das Edelmetall Ag ist.

9. Gassensor-Array nach Anspruch 2,
dadurch **gekennzeichnet,**
daß das katalytisch aktive Material (4) ein Nebengruppen-Metalloxid ist.

10. Gassensor-Array nach Anspruch 9,
dadurch **gekennzeichnet,**
daß das Nebengruppenmetalloxid Nb₂O₃ ist.

11. Gassensor-Array nach Anspruch 9,
dadurch **gekennzeichnet,**
daß das Nebengruppenmetalloxid Fe₂O₃ ist.

12. Gassensor-Array nach Anspruch 9,
dadurch **gekennzeichnet,**
daß das Nebengruppen-Metalloxid ZrO₂ ist.

13. Gassensor-Array nach Anspruch 9,
dadurch **gekennzeichnet,**
daß das Nebengruppen-Metalloxid TiO₂ ist.

14. Gassensor-Array nach einem der Ansprüche 2 bis 13,
dadurch **gekennzeichnet,**
daß das katalytisch aktive Material (4) auf die Oberfläche der gassensitiven Ga₂O₃-Dünnschicht (3) in Form einer zweiten Phase aufgebracht ist.

15. Gassensor-Array nach einem der Ansprüche 2 bis 13,
dadurch **gekennzeichnet,**
daß sich das katalytisch aktive Material (4) in Form einer zweiten Phase innerhalb der gassensitiven Ga₂O₃-Dünnschicht befindet.

16. Gassensor-Array nach einem der vorhergehenden Ansprüche,
dadurch **gekennzeichnet,**
daß bei zumindest einem der Einzelsensorelemente die ursprünglich n-halbleitende Ga₂O₃-Dünnschicht (3) zur Erfassung oxidierender Gase durch Dotierung zu einer p-halbleitenden Dünnschicht umgewandelt ist.

17. Gassensor-Array nach Anspruch 16,
dadurch **gekennzeichnet,**
daß das Dotierungsmaterial MgO ist.

18. Gassensor-Array nach einem der vorhergehenden Ansprüche,
dadurch **gekennzeichnet,**
daß die Einzelsensorelemente unterschiedliche Schichtmorphologien aufweisen.

19. Gassensor-Array nach einem der Ansprüche 1-18,
dadurch **gekennzeichnet,**
daß die Betriebstemperaturen für die Einzelsensorelemente gleich sind.

20. Gassensor-Array nach einem der Ansprüche 1 bis 18,
dadurch **gekennzeichnet,**
daß für die Erfassung von O₂ die Betriebstemperatur bei 950°C und für die Erfassung von reduzieren den Gasen bei 600°C liegt.

21. Gassensor-Array nach einem der Ansprüche 1 bis 20,
dadurch **gekennzeichnet**,
daß auf der sensorabgewandten Seite des Trägerkörpers wenigstens eine Heizstruktur vorgesehen ist.

22. Gassensor-Array nach einem der Ansprüche 1 bis 18, 20 oder 21,
dadurch **gekennzeichnet,**
daß zumindest durch eine oder mehrere Wärmebarrieren thermisch isolierte Bereiche des Trägerkörpers vorgesehen sind und daß jedem dieser Bereiche eine eigene Heizstruktur zugeordnet ist, so daß die unterschiedlichen Betriebstemperaturen bereitgestellt sind.

23. Gassensor-Array nach Anspruch 22,
dadurch **gekennzeichnet,**
daß die Heizstrukturen in Reihe geschaltet sind.

24. Gassensor-Array nach Anspruch 22,
dadurch **gekennzeichnet,**
daß die Heizstrukturen parallel geschaltet sind.

25. Gassensor-Array nach einem der Ansprüche 1 bis 18 oder 20,
dadurch **gekennzeichnet**,
daß die unterschiedlichen Betriebstemperaturen durch eine Struktur, die sich aus einer Kombination der Anordnungen gemäß den Ansprüchen 21 bis 24 ergibt, erzeugt werden.

26. Gassensor-Array nach einem der Ansprüche 22 bis 25,
dadurch **gekennzeichnet,**
daß die Wärmebarriere in Form einer Einsenkung in dem Trägerkörper ausgebildet ist.

27. Gassensor-Array nach einem der Ansprüche 22 bis 25,
dadurch **gekennzeichnet,**
daß die Wärmebarriere in Form eines Spalts in dem Trägerkörper ausgebildet ist.

28. Gassensor-Array nach einem der Ansprüche 1 bis 27,
dadurch **gekennzeichnet,**
daß der Trägerkörper aus einem aluminiumfreien Keramikmaterial besteht.

29. Gassensor-Array nach einem der Ansprüche 1 bis 27,
dadurch **gekennzeichnet,**
daß der Trägerkörper aus Quarzglas besteht.

30. Gassensor-Array nach einem der Ansprüche 1 bis 27,
dadurch **gekennzeichnet,**
daß der Trägerkörper aus Silizium mit zumindest einer elektrisch isolierenden Schicht besteht.

## Claims

1. Gas sensor array for the detection of individual gas components in a gas mixture, comprising a multiplicity of individual sensor elements based on semi-conducting metal oxides, the individual sensor elements each being applied to an electrically nonconducting substrate, the array being provided with a contact-electrode arrangement for measuring the electrical conductivities, a heating arrangement for heating at a predetermined operating temperature, a protective envelope which protects the array against external mechanical effects and a mounting base, predetermined individual operating temperatures being assigned to the individual sensor elements, and differences being formed between the respective sensor signals for the detection of the individual gas components, which differences are fed to a processing unit, characterized in that at least some of the individual sensor elements are based on β-Ga₂O₃ thin films (3) and in that all the individual sensor elements are provided in a planar arrangement on a common substrate (1).

2. Gas sensor array according to Claim 1, characterized in that at least one of the individual sensor elements is provided with at least one finely dispersed, catalytically active material (4) whose action is stable in the prevailing ambient conditions concerned.

3. Gas sensor array according to Claim 2, characterized in that the catalytically active material (4) is a noble metal.

4. Gas sensor array according to Claim 3, characterized in that the catalytically active material (4) is an alloy with at least one noble metal.

5. Gas sensor array according to Claim 3, characterized in that the noble metal is Pt.

6. Gas sensor array according to Claim 3, characterized in that the noble metal is Pd.

7. Gas sensor array according to Claim 3, characterized in that the noble metal is Rh.

8. Gas sensor array according to Claim 3, characterized in that the noble metal is Ag.

9. Gas sensor array according to Claim 2, characterized in that the catalytically active material (4) is a subgroup metal oxide.

10. Gas sensor array according to Claim 9, characterized in that the subgroup metal oxide is Nb₂O₃.

11. Gas sensor array according to Claim 9, characterized in that the subgroup metal oxide is Fe₂O₃.

12. Gas sensor array according to Claim 9, characterized in that the subgroup metal oxide is ZrO₂.

13. Gas sensor array according to Claim 9, characterized in that the subgroup metal oxide is TiO₂.

14. Gas sensor array according to one of Claims 2 to 13, characterized in that the catalytically active material (4) is applied to the surface of the gas-sensitive Ga₂O₃ thin film (3) in the form of a second phase.

15. Gas sensor array according to one of Claims 2 to 13, characterized in that the catalytically active material (4) is in the form of a second phase inside the gas-sensitive Ga₂O₃ thin film.

16. Gas sensor array according to one of the preceding claims, characterized in that, in the case of at least one of the individual sensor elements, the originally n-type semiconducting Ga₂O₃ thin film (3) is converted into a p-type semiconducting thin film by doping for the purpose of detecting oxidizing gases.

17. Gas sensor array according to Claim 16, characterized in that the doping material is MgO.

18. Gas sensor array according to one of the preceding claims, characterized in that the individual sensor elements have different film morphologies.

19. Gas sensor array according to one of Claims 1-18, characterized in that the operating temperatures for the individual sensor elements are identical.

20. Gas sensor array according to one of Claims 1 to 18, characterized in that, for the detection of O₂, the operating temperature is 950°C and, for the detection of reducing gases, it is 600°C.

21. Gas sensor array according to one of Claims 1 to 20, characterized in that at least one heating structure is provided on that side of the carrier body which is remote from the sensor.

22. Gas sensor array according to one of Claims 1 to 18, 20 or 21, characterized in that thermally isolated regions of the carrier body are provided at least by one or more heat barriers and in that a separate heating structure is assigned to each of said regions so that the different operating temperatures are provided.

23. Gas sensor array according to Claim 22, characterized in that the heating structures are connected in series.

24. Gas sensor array according to Claim 22, characterized in that the heating structures are connected in parallel.

25. Gas sensor array according to one of Claims 1 to 18 or 20, characterized in that the different operating temperatures are generated by a structure which results from a combination of the arrangements according to Claims 21 to 24.

26. Gas sensor array according to one of Claims 22 to 25, characterized in that the heat barrier is constructed in the form of a depression in the carrier body.

27. Gas sensor array according to one of Claims 22 to 25, characterized in that the heat barrier is constructed in the form of a gap in the carrier body.

28. Gas sensor array according to one of Claims 1 to 27, characterized in that the carrier body comprises an aluminium-free ceramic material.

29. Gas sensor array according to one of Claims 1 to 27, characterized in that the carrier body comprises quartz glass.

30. Gas sensor array according to one of Claims 1 to 27, characterized in that the carrier body comprises silicon having at least one electrically insulating film.

## Revendications

1. Réseau de détecteurs de gaz pour la détection de constituants individuels de gaz dans un mélange gazeux, constitué par une multiplicité d'éléments détecteurs individuels réalisés à base d'oxydes métalliques semiconducteurs, et dans lequel les éléments détecteurs individuels sont déposés respectivement sur un substrat non conducteur du point de vue électrique, le réseau est équipé d'un dispositif d'électrodes de contact servant à mesurer la conductivité électrique, d'un dispositif de chauffage servant à réaliser un chauffage pour une température de fonctionnement prédéterminée, d'une douille de protection qui protège le réseau vis-à-vis d'influences mécaniques extérieures, et d'un socle de fixation, et dans lequel des températures individuelles prédéterminées de fonctionnement sont associées aux éléments détecteurs individuels et, pour la détection des différents constituants du gaz, des différences sont formées entre les signaux respectifs des détecteurs, différences qui peuvent être envoyées à une unité de traitement,
caractérisé par le fait qu'au moins une partie des éléments détecteurs individuels est réalisée sur la base de couches minces (3) de β-Ga₂O₃ et que tous les éléments détecteurs individuels sont prévus, selon une disposition planar, sur un substrat commun (1).

2. Réseau de détecteurs de gaz suivant la revendication 1, caractérisé par le fait qu'au moins l'un des éléments détecteurs individuels est pourvu au moins d'un matériau finement dispersé, actif du point de vue catalytique (4), qui fournit une action stable dans les conditions d'environnement existantes considérées.

3. Réseau de détecteurs de gaz suivant la revendication 2, caractérisé par le fait que le matériau actif du point de vue catalytique (4) est un métal précieux.

4. Réseau de détecteurs de gaz suivant la revendication 3, caractérisé par le fait que le matériau actif du point de vue catalytique (4) est un alliage comportant au moins un métal précieux.

5. Réseau de détecteurs de gaz suivant la revendication 3, caractérisé par le fait que le métal précieux est du Pt.

6. Réseau de détecteurs de gaz suivant la revendication 3, caractérisé par le fait que le métal précieux est du Pd.

7. Réseau de détecteurs de gaz suivant la revendication 3, caractérisé par le fait que le métal précieux est du Rh.

8. Réseau de détecteurs de gaz suivant la revendication 3, caractérisé par le fait que le métal précieux est du Ag.

9. Réseau de détecteurs de gaz suivant la revendication 2, caractérisé par le fait que le matériau actif du point de vue catalytique (4) est un oxyde métallique d'un groupe secondaire.

10. Réseau de détecteurs de gaz suivant la revendication 9, caractérisé par le fait que l'oxyde métallique du groupe secondaire est du Nb₂O₃.

11. Réseau de détecteurs de gaz suivant la revendication9, caractérisé par le fait que l'oxyde métallique du groupe secondaire est du Fe₂O₃.

12. Réseau de détecteurs de gaz suivant la revendication9, caractérisé par le fait que l'oxyde métallique du groupe secondaire est du ZrO₂.

13. Réseau de détecteurs de gaz suivant la revendication9, caractérisé par le fait que l'oxyde métallique du groupe secondaire est du TiO₂.

14. Réseau de détecteurs de gaz suivant l'une des revendications 2 à 13, caractérisé par le fait que le matériau actif du point de vue catalytique (4) est déposé sur la surface de la couche mince en Ga₂O₃ (3) sensible au gaz, sous la forme d'une seconde phase.

15. Réseau de détecteurs de gaz suivant l'une des revendications 2 à 13, caractérisé par le fait que le matériau actif du point de vue catalytique (4) est présent sous la forme d'une seconde phase à l'intérieur de la couche mince en Ga₂O₃ sensible au gaz.

16. Réseau de détecteurs de gaz suivant l'une des revendications précédentes, caractérisé par le fait que dans le cas d'au moins l'un des éléments détecteurs individuels, la couche mince de Ga₂O₃ (3) initialement semiconductrice de type n est convertie, par dopage, pour la détection de gaz oxydants, en une couche mince semiconductrice du type p.

17. Réseau de détecteurs de gaz suivant la revendication 16, caractérisé par le fait que le matériau de base est du MgO.

18. Réseau de détecteurs de gaz suivant l'une des revendications précédentes, caractérisé par le fait que les éléments détecteurs individuels possèdent des morphologies de couches différentes.

19. Réseau de détecteurs de gaz suivant l'une des revendications 1-18, caractérisé par le fait que les températures de fonctionnement sont identiques pour les éléments détecteurs individuels.

20. Réseau de détecteurs de gaz suivant l'une des revendications 1 à 18, caractérisé par le fait que pour la détection de O₂, la température de fonctionnement est égale à 950°C et que pour la détection de gaz réducteurs, elle est égale à 600°C.

21. Réseau de détecteurs de gaz suivant l'une des revendications 1 à 20, caractérisé par le fait qu'au moins une structure chauffante est prévue sur le côté du corps de support, tourné à l'opposé des détecteurs.

22. Réseau de détecteurs de gaz suivant l'une des revendications 18, 20 ou 21, caractérisé par le fait que des zones du corps de support, thermiquement isolées, sont prévues au moins grâce à la présence d'une ou de plusieurs barrières thermiques et qu'à chacune de ces zones est associée une structure chauffante particulière de sorte que l'on obtient des différences de température de fonctionnement.

23. Réseau de détecteurs de gaz suivant la revendication 22, caractérisé par le fait que les structures chauffantes sont branchées en série.

24. Réseau de détecteurs de gaz suivant la revendication 22, caractérisé par le fait que les structures chauffantes sont branchées en parallèle.

25. Réseau de détecteurs de gaz suivant l'une des revendications 1 à 18 ou 20, caractérisé par le fait que les différentes températures de fonctionnement sont produites par une structure qui résulte d'une combinaison du dispositif selon les revendications 21 à 24.

26. Réseau de détecteurs de gaz suivant l'une des revendications 22 à 25, caractérisé par le fait que la barrière thermique est réalisée sous la forme d'un renfoncement dans le corps de support.

27. Réseau de détecteurs de gaz suivant l'une des revendications 22 à 25, caractérisé par le fait que la barrière thermique est réalisée sous la forme d'une fente dans le corps de support.

28. Réseau de détecteurs de gaz suivant l'une des revendications 1 à 27, caractérisé par le fait que le corps de support est réalisé en un matériau céramique exempt d'aluminium.

29. Réseau de détecteurs de gaz suivant l'une des revendications 1 à 27, caractérisé par le fait que le corps de support est réalisé en verre quartzeux.

30. Réseau de détecteurs de gaz suivant l'une des revendications 1 à 27, caractérisé par le fait que le corps de support est réalisé en silicium comportant au moins une couche électriquement isolante.
